# EUROPEAN PATENT APPLICATION

(11) **EP 2 897 067 A1**
(43) Date of publication of application: **22.07.2015**
(21) Application number: 14193463.8
(22) Date of filing: 17.11.2014
(51) Int. Cl.: G06F 19/00, A63B 24/00

(54) **A guiding intermittent aerobic exercise system and method**

(30) Priority: 13.12.2013 TW 102146301
(71) Applicant: Advanced Mediwatch Co., Ltd., Taipei City (TW)
(72) Inventor: Lin, Sung-Lien, New Taipei City (TW); Lin, Shang-Ting, Taipei City (TW)
(74) Representative: Zacco Sweden AB

(57) **Abstract**

The present invention provides a guiding aerobic exercise system and method for guiding a user to perform intermittent aerobic interval exercise with moderate or high intensity. The invention surprisingly demonstrates that following the guidance of the system and method of the invention, the user can improve metabolic conditions to attain a healthier body.

## Description

### Field of the Invention

The invention relates to a guiding system for an intermittent aerobic exercise system and method. Particularly, the invention provides a device guiding an intermittent aerobic exercise system in intervals of moderate or strong intensity.

### Background of the Invention

Aerobic metabolism refers to a method of producing energy in a body by a process requiring oxygen. Whenever the available amount of oxygen-based energy fails to meet the demand, an anaerobic state occurs where energy is produced by a process that does not require oxygen, but may result in an oxygen debt.

Aerobic exercise is physical exercise of moderate to high intensity over a long period of time, which mainly uses the energy generated from aerobic metabolism and consumes fat and glycogen in the body. Aerobic exercise can improve heart and lung capacity, and it has also been widely used for weight loss because it can burn fat. Aerobic exercise includes, for example, jogging, cycling, boating, skiing, skating, jumping rope, swimming, walking, rhythmic gymnastics, climbing and so on. The general definition of aerobic exercise such as jogging is exercise maintaining the same heart rate (HR) intensity for more than 15 minutes. The continuous moderate endurance (CME) refers to the heart rate maintained at the same heartbeat intensity for 15 minutes or maintained at moderate intensity (50 to 70% of the maximum heart rate). Besides high intensity interval training (HIIT) including intervals of warm up and high intensity (75% to 100% of the maximum heart rate) is a precise workout regimen for health. There is a need in how to guide users to conduct aerobic exercise during exercise without producing too much oxygen debt. In sports and exercise testing, the Borg Rating of Perceived Exertion (RPE) Scale measures perceived exertion. There is grossly ranking of 6 to 7 easy 8 to 9 very light 10 to 11 light 12 to 13 somewhat hard 14 to 15 hard 16 to 17 very hard 18 to 19 extremely hard 20 maximal exertion. The score of 6 to 20 is to follow the general HR of a "healthy" adult by multiple by 10. While the two parameters are not consistent during exercise, physical problem may be concerned during exercise. Double-checking of the two parameters during exertion workout is crucial for health and safety.

US 7,828,697 relates to a portable personal training device including an operable geographic location determining component to determine the location of the device, where a housing having a first portion and a second portion coupled to the first portion is at an angle, and a strap operable to secure the housing to a user's wrist such that the first portion is operable to be positioned on top around the wrist and the second portion is operable to be positioned offset from the top of the wrist. Such configuration facilitates both the wearing and the operation of the device. US 7,789,802 provides a physical training device using GPS data to assist a user in reaching their performance goals and completing training sessions by tracking their performance, by communicating progress including progress relative to user-defined goals, by communicating navigation directions and waypoints, and by storing and analyzing training session statistics. US 8,033,959 further provides a portable fitness monitoring system that includes: a portable fitness monitoring device; a sensor that communicates with the portable fitness monitoring device to sense the performance parameters during physical activity conducted by the user and communicates the performance parameter data to the dedicated portable fitness monitoring device; a music device directly coupled to the portable fitness monitoring device; and an audio output device directly coupled to the portable fitness monitoring device, wherein music is transmitted from the portable music device to the audio output device through the portable fitness monitoring device.

US20110151420A1 provides a convenient device (such as a mobile phone) providing a user interface for a system that generates personalized exercise programs and guides users through the exercises in a generated program. US20110077130A1 provides an exercise assistance device that includes: a first acquisition unit which acquires plural pieces of exercise scene information representing a scene when an exercise motion is performed and plural pieces of motion information representing the exercise motions, the plural pieces of exercise scene information being associated with the plural pieces of motion information; a second acquisition unit which acquires plural pieces of exercise order information which describes an order to perform the exercise motions corresponding to the exercise scene information; a first selection unit which selects one of the pieces of exercise order information acquired by the second acquisition unit; a decision unit which decides, from plural pieces of motion information, the motion information corresponding to the exercise scene information included in the exercise order information selected by the first selection unit; and a generation unit which generates a display signal to display the motion information decided by the decision unit on a display unit. Taiwan Patent NOs 403668 and 403669 detect body fat rate prior to exercise and determine a target body fat rate; an exercise program should be decided to determine what load level, what frequency of exercise, and what continuous time for the exercise so that the load of exercise meets the program. Although the above prior art teaches guiding aerobic exercise system, it can only reach the effect of common aerobic exercise and cannot guide user to perform aerobic exercise that can improve metabolism.

Current exercise programs have not been subjected to precise evaluation, so whether the exercise can improve metabolism and aging cannot be ascertained. Therefore, there is a need to develop an aerobic exercise system that can improve metabolism and is beneficial to health.

### Summary of the Invention

If a high intensity maximum heart rate (75% to 100%) is used as a target interval during exercise, it is effective in resisting metabolic diseases; however, the exercise cannot be continuously performed over a long period of time. Moreover, it needs a graded elevation and a recovery interval with a lower intensity maximum heart rate (40% to 70%) between two high intensity intervals. The graded intervals having high and low intensities for fixed times refer to aerobic interval training or high intensity interval training, which have better efficacy in reducing blood pressure and blood sugar, strengthening the heart and burning fat than aerobic exercise of continuous moderate endurance. Accordingly, the invention provides a system and method that guides a user to perform an intermittent aerobic exercise in moderate or strong intensity interval(s) preferably with a warm-up period for gradually elevating HR intensity to a target zone, and the inventor surprisingly found that the user performing the exercise guided by the system and method of the invention can improve metabolism and thus attain better health.

### Brief Description of the Drawing

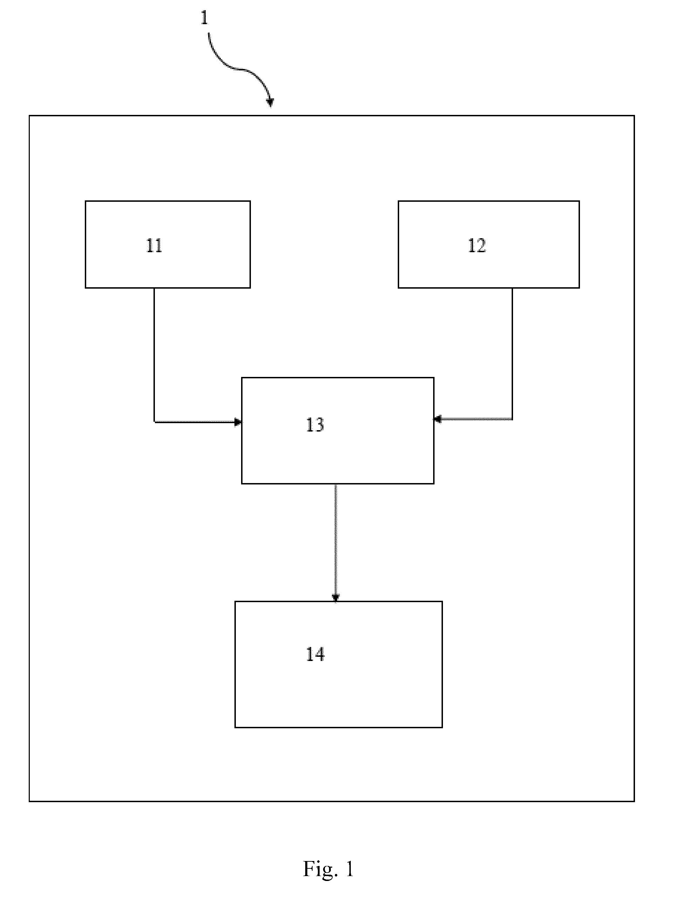

Figure 1 shows a block diagram illustrating elements of the intermittent aerobic exercise system as one embodiment of the invention.

### Detailed Description of the Invention

In one aspect, the invention provides an intermittent aerobic exercise system, comprising:
(a) a heart rate sensor unit for detecting a heart rate reference of a user;
(b) a memory unit for saving one or plural target heart rate reference intervals, said one interval comprising a target heart rate reference and a time period of maintaining the target heart rate reference;
(c) a process unit connecting the heart rate sensor unit and a guiding unit, said process unit receiving the heart rate reference detected by the heart rate sensor unit and sending a signal of directing to the target heart rate reference interval according to the target heart rate reference interval saved in the memory unit; and
(d) a guiding unit connecting the process unit, receiving the signal from the process unit and providing a direction signal to guide the user to adjust exercise intensity following the target heart rate reference interval.

In another aspect, the invention provides a method for guiding an intermittent aerobic exercise, comprising the following steps:
(a) providing a heart rate sensor unit for detecting a heart rate reference of a user;
(b) setting one or plural target heart rate reference intervals, said one interval comprising a target heart rate reference and a time period of maintaining the target heart rate;
(c) receiving the heart rate reference detected by the heart rate sensor unit to a process unit and sending a signal of directing to the target heart rate reference interval according to the target heart rate reference interval saved in the memory unit; and
(d) receiving and displaying the signal from the process unit to guide the user to adjust exercise intensity following the target heart rate reference interval.

In one embodiment, the heart rate sensor unit of the guiding aerobic exercise system of the invention is used to detect the heart rate reference of the user. The heart rate reference can be detected based on pulse, heart rate or heart intensity. The pulse is the impulse of artery and the impulse is caused by cardiac impulse. Under normal condition, the pulse equals to heart beats, so pulse or heart rate is the frequency of heart's blood output per minute.

The heart rate (HR) intensity refers to exercise intensity calculated from heart rate. Various equations or formulae for determining the HR intensity can be used in the invention. In one example, HR intensity equals to HRmax multiplied by (220-age), which can be calculated based on the following equation: (220-age) x %.

In the above equation, HRmax is the maximum heart rate that of an individual under safe exercise pressure and depends to age. Other calculations of predicted maximum heart rate based on age are provided by Hirofumi Tanake et al. (J of Am College of Cardiology 2001: 153-6).

In one embodiment, the heart rate sensor unit is an internal or external rhythm.

In one embodiment, the memory unit of the aerobic exercise system of the invention is used for saving one or plural target heart rate reference intervals, said interval comprising a target heart rate reference and a time period of maintaining the target heart rate. The target heart rate reference interval includes any length of time period of maintaining the target heart rate reference and a target heart rate reference with any heart rate intensity. In one embodiment, the arrangement of the plural target heart rate reference intervals can be gradually increased or decreased according to heart rate intensity or repeated with identical heart rate intensity. In one embodiment, the target heart rate reference interval in the memory unit can be originally set in the system or artificially set by the user. Since the health effects generated by exercise can be accumulated; therefore, the system and method of the invention can improve metabolism and aging. The invention can use various combinations of exercise intensity and time period that can achieve intermittent aerobic exercise to set target heart rate reference intervals.

In one embodiment, the target heart rate reference intervals are set based on heart rate intensity. Preferably, the target heart rate reference interval is preceded by a warm-up period. Preferably, a target heart rate reference interval of the invention includes an aerobic exercise with a target heart rate reference of about 70% to about 100% of maximum heart rate for a time period of at least several minutes, preferably following a heart rate recovery interval guiding of a heart rate intensity with about 40% to about 70% of maximum heart rate. Preferably, after the above intermittent aerobic exercise, another interval includes heart rate intensity with about 70% to about 100% of maximum heart rate for a time period of several minutes, following a heart rate recovery interval guiding of a heart rate intensity with about 40% to about 70% of maximum heart rate. Preferably, the target heart rate reference interval further includes a warm-up period and a target heart rate reference with a heart rate for a time period is set for the warm-up period. As such, several intervals can be repeated (preferably, 2 to 20 times). Elevation of the heart rate reference (i.e., heart rare intensity) is guided in graded steps for user to adapt every level. Preferably, the target heart rate reference interval includes in sequence, a section with a target heart rate reference of about 70% of maximum heart rate for a time period of about 8 to about 12 minutes (preferably about 10 minutes), four sections with a target heart rate reference of about 90% of maximum heart rate for a time period of about 2 to about 6 minutes (preferably about 4 minutes) and an intermission between two sections with a target heart rate reference of about 70% of maximum heart rate for a time period of about 1.5 to about 5 minutes (preferably about 3 minutes), and a recovery section with a time period of about 3 to about 8 minutes (preferably about 5 minutes). In one embodiment, the target heart rate reference intervals include the following intervals: a target heart rate reference of about 70% of maximum heart rate for a time period of about 10 minutes, a target heart rate reference of about 90% of maximum heart rate for a time period of about 4minutes, a target heart rate reference of about 70% of maximum heart rate for a time period of about 3 minutes, a target heart rate reference of about 90% of maximum heart rate for a time period of about 4 minutes, a target heart rate reference of about 70% of maximum heart rate for a time period of about 3 minutes, a target heart rate reference of about 90% of maximum heart rate for a time period of about 4 minutes, a target heart rate reference of about 70% of maximum heart rate for a time period of about 3 minutes, a target heart rate reference of about 90% of maximum heart rate for a time period of about 7 minutes and a target heart rate reference of about 70% of maximum heart rate for a time period of about 5 minutes. Preferably, the initiating target heart rate reference interval further includes a warm-up period. Preferably, after a target heart rate reference interval, a heart rate recovery interval guiding of a heart rate intensity with about 40% to about 70% of maximum heart rate can be followed.

In another embodiment, the target heart rate reference intervals are set based on peak oxygen uptake. The "peak oxygen uptake" refers to the maximum rate of oxygen consumption (VO2peak) as measured during an exercise. Preferably, the target heart rate reference interval includes in sequence, a section with 70% VO2peak for about 1.5 to 6 minutes (preferably about 3 minutes) and a section with ≤ 40% VO2peak for about 1.5 to 6 minutes (preferably about 3 minutes). Preferably, the above interval can be repeated three to seven times (preferably five times).

In one embodiment, the system of the invention further comprises an interface of converting a perceived exertion of a user rated by Borg scale to a target heart rate reference. Alternatively, the heart rate sensor unit can be replaced by an interface of converting a perceived exertion of a user rated by Borg scale to a target heart rate reference. The consistency of the heart rate reference detected by the heart rate sensor unit and that converted from Borg scale are continuously checked during exercise. If the heart rate reference detected by the heart rate sensor unit is inconsistent with that converted from Borg scale, it represents that the user may has physical problem.. Borg scale measures perceived exertion according to the use's feeling in heartbeat, respiration, sweating and muscle fatigue ("Perceived exertion as an indicator of somatic stress". Scandinavian journal of rehabilitation medicine 2 (2): 92-98; Borg scale for measuring perceived exertion (Borg (1998) Human kinetics, Champaign, Ill.; Nybo (2003) Med Sci Sports Exerc, 35, 589-594). The scale ranges from 6 to 20, where 6 means no exertion at all, 7 and 8 mean extremely light, 9 and 10 mean very light, 11 and 12 mean light, 13 and 14 mean somewhat hard, 15 and 16 mean hard/heavy, 17 and 18 mean very hard, 19 means extremely hard and 20 means maximal exertion. The heart rate can be obtained by multiplying Borg scale by 10.

The process unit of the intermittent aerobic exercise system of the invention connects the heart rate sensor unit and a guiding unit and it receives the heart rate reference detected from the heart rate sensor unit and sends a signal of directing to the target heart rate reference interval according to the target heart rate reference interval saved in the memory unit so that the user can adjust exercise intensity following the target heart rate reference interval. Elevation of the heart rate intensity is guided in graded steps for user to adapt every level of the target intensity. In another embodiment, the process unit can transmit a signal to a server through internet or global positioning system (GPS), receives a signal in order to save heart rate reference of the user and change target heart rate reference interval in the memory unit to employ the process unit to provide instant information guiding the user to adjust exercise intensity.

The guiding unit of the connecting the process unit to receive the signal from the process unit and providing a direction signal to guide the user to adjust exercise intensity following the target heart rate reference interval. In one embodiment, the guiding unit guides the exercise intensity by stepped increased or decreased.

In another embodiment, the guiding unit is a display showed by light, color, voice, vibration, icon or words to guide the user to adjust exercise intensity. Preferably, the guiding unit is a light-emitting diode guiding a user to adjust exercise intensity by light. Preferably, the guiding unit is a vibrating motor guiding a user to adjust exercise intensity by vibration. Preferably, the guiding unit is an amplifier guiding a user to adjust exercise intensity by voice. Preferably, the guiding unit is a visual display guiding a user to adjust exercise intensity by color; more preferably, the numbers and types of color levels of the visual display can be designed according to the objective and necessity of the manufactures. For example, the color levels can be shown with fixed colors and/or blinking colors and the numbers of the color levels may range from 1 to 20 color levels, 1 to 10 color levels, 1 to 7 color levels, or 1 to 5 color levels. For example, 5 color levels in combination with a fixed and/or a blinking color can be used for display. For example, the color levels range from blue to red, so that from the lowest to the highest sequence are blue (B) 151, green (G) 152, yellow (Y) 153, orange (O) 154 and red (R) 155 and the blinking color of the color level is denoted with the symbol "*."

In one embodiment, the guiding unit connects to an exercise device for adjusting velocity of the exercise device to guide the user to adjust exercise intensity so that the user follows the target heart rate reference interval. Preferably, the exercise device is a treadmill or ergometer; the guiding unit connects to the treadmill to automatically adjust race velocity to guide a user to adjust exercise intensity following the target heart rate reference interval.

In one embodiment, the guiding unit sends a signal (such as beat or musical rhythm) to guide a user to adjust exercise motion. After heart rate intensity follows the target heart rate reference intensity, if the heart rate intensity of the user is lower (or higher) than the target heart rate reference intensity, the process unit can further send a signal to the guiding unit so that the guiding unit sends a further instruction signal to guide the user to enlarge or reduce motion to achieve the target heart rate reference. For examiner, the motion of step, leg list and/or hand waving can be enlarged.

Now with reference to the embodiment as illustrated in the accompanying drawings of the present invention to the present invention will be described in detail. "One embodiment", "an example embodiment", etc. refer to directions, an embodiment may include a particular feature, structure, or characteristic of the described, but each of the embodiments may not necessarily include the particular feature, structure, or characteristic. In addition, these phrases may not necessarily represent the same embodiment. Further, when a combination of the described embodiments a particular feature, structure, or characteristic is that a particular feature, structure, or characteristic lines in the knowledge of the skilled person skilled in the connection with other embodiments to affect this feature, structure, or characteristic, regardless of whether clear description.

Referring to Figure 1 as one embodiment, the guiding aerobic exercise system 1 includes a heart rate sensor unit 11, a memory unit 12, a process unit 13 and a guiding unit 14. The heart rate sensor unit 11 detects pulse, heart rate or heart rate intensity of a user. The memory unit 12 saves one or plural target heart rate reference interval; for example, the interval includes in sequence, a section with 70% of maximum heart rate for 10 minutes, four sections with 90% of maximum heart rate for 4 minutes and an intermission between two sections with 70% of maximum heart rate for 3 minutes, and a recovery section for 5 minutes. The heart rate sensor unit 11 transmits heart rate reference to the process unit 13 and the process unit 3 connects the heart rate sensor unit 11 and the memory unit 12. After the process unit 13 receives the heart rate reference from the heart rate sensor unit 11, it sends a signal to the guiding unit 14 according to the saved target heart rate reference interval so that the guiding unit 14 provides a signal to the user to adjust exercise intensity following the target heart rate reference interval.

## Claims

1. An intermittent aerobic exercise system, comprising:
(a) a heart rate sensor unit for detecting a heart rate reference of a user;
(b) a memory unit for saving one or plural target heart rate reference intervals, said one interval comprising a target heart rate reference and a time period of maintaining the target heart rate reference;
(c) a process unit connecting the heart rate sensor unit and a guiding unit, said process unit receiving the heart rate reference detected by the heart rate sensor unit and sending a signal of directing to the target heart rate reference interval according to the target heart rate reference interval saved in the memory unit; and
(d) a guiding unit connecting the process unit, receiving the signal from the process unit and providing a direction signal to guide the user to adjust exercise intensity following the target heart rate reference interval.

2. The system of Claim 1, wherein the heart rate reference is pulse, heart rate or heart intensity and the heart rate sensor unit is an internal or external rhythm.

3. The system of Claim 1, which further comprises an interface of converting a perceived exertion of a user rated by Borg scale to a target heart rate reference, optionally, the consistency of the heart rate reference detected by the heart rate sensor unit and that converted from Borg scale are continuously checked during exercise.

4. The system of Claim 1, wherein the heart rate sensor unit can be replaced by an interface of converting a perceived exertion of a user rated by Borg scale to a target heart rate reference.

5. The system of Claim 1, wherein the memory unit saves plural target heart rate reference intervals, optionally, the arrangement of the plural target heart rate reference intervals can be gradually increased or decreased according to heart rate intensity or repeated with identical heart rate intensity.

6. The system of Claim 1, wherein the target heart rate reference interval in the memory unit comprises a target heart rate reference and a time period of maintaining the target heart rate reference.

7. The system of Claim 1, wherein the target heart rate reference interval in the memory unit can be originally set in the system or artificially set by the user.

8. The system of Claim 1, wherein the target heart rate reference intervals are set based on heart rate intensity and the target heart rate reference interval includes any length of time period of maintaining the target heart rate reference and a target heart rate reference with any heart rate intensity.

9. The system of Claim 1, wherein the target heart rate reference interval includes an aerobic exercise with a target heart rate reference of about 70% to about 100% of maximum heart rate for a time period of at least several minutes, preferably following a heart rate recovery interval guiding of a heart rate intensity with about 40% to about 70% of maximum heart rate.; optionally following additional one or more the identical interval(s).

10. The system of Claim 1, wherein the target heart rate reference interval includes in sequence, a section with a target heart rate reference of about 70% of maximum heart rate for a time period of about 8 to about 12 minutes (preferably about 10 minutes), four sections with a target heart rate reference of about 90% of maximum heart rate for a time period of about 2 to about 6 minutes (preferably about 4 minutes) and an intermission between two sections with a target heart rate reference of about 70% of maximum heart rate for a time period of about 1.5 to about 5 minutes (preferably about 3 minutes), and a recovery section with a time period of about 3 to about 8 minutes (preferably about 5 minutes).

11. The system of Claim 1, wherein the target heart rate reference intervals include the following intervals: a target heart rate reference of about 70% of maximum heart rate for a time period of about 10 minutes, a target heart rate reference of about 90% of maximum heart rate for a time period of about 4minutes, a target heart rate reference of about 70% of maximum heart rate for a time period of about 3 minutes, a target heart rate reference of about 90% of maximum heart rate for a time period of about 4 minutes, a target heart rate reference of about 70% of maximum heart rate for a time period of about 3 minutes, a target heart rate reference of about 90% of maximum heart rate for a time period of about 4 minutes, a target heart rate reference of about 70% of maximum heart rate for a time period of about 3 minutes, a target heart rate reference of about 90% of maximum heart rate for a time period of about 7 minutes and a target heart rate reference of about 70% of maximum heart rate for a time period of about 5 minutes.

12. The system of Claim 1, wherein the initiating target heart rate reference interval is preceded by a warm-up period.

13. The system of Claim 1, wherein the target heart rate reference interval includes in sequence, a section with 70% VO₂ peak for 3 minutes and a section with ≤ 40% VO₂peak for 3 minutes; optionally, interval can be repeated five times for 30 minutes in total.

14. The system of Claim 1, wherein the guiding unit retrieves the heart rate reference through the heart rate sensor unit and guides to direct to the target heart rate reference interval, optionally the guiding unit guides the exercise intensity by stepped or gradually increasing or decreasing target heart rate reference.

15. The system of Claim 1, wherein the guiding unit is a display showed by light, color, voice, vibration, icon or words to guide the user to adjust exercise intensity.

16. The system of Claim 156, wherein the color levels can be shown with fixed colors and/or blinking colors and the numbers of the color levels may range from 1 to 10 color levels.

17. A method for guiding an intermittent aerobic exercise by an intermittent aerobic exercise system according to Claim 1, comprising the following steps:
(a) providing a heart rate sensor unit for detecting a heart rate reference of a user;
(b) setting one or plural target heart rate reference intervals, said one interval comprising a target heart rate reference and a time period of maintaining the target heart rate;
(c) receiving the heart rate reference detected by the heart rate sensor unit to a process unit and sending a signal of directing to the target heart rate reference interval according to the target heart rate reference interval saved in the memory unit; and
(d) receiving and displaying the signal from the process unit to guide the user to adjust exercise intensity following the target heart rate reference interval.
